# EUROPEAN PATENT SPECIFICATION

(11) **EP 3 300 704 B1**
(45) Date of publication and mention of the grant of the patent: **02.03.2022**
(21) Application number: 17193713.9
(22) Date of filing: 28.09.2017
(51) Int. Cl.: A61F 9/007, A61M 1/00, A61M 39/22

(54) **OPHTHALMIC APPARATUS**
OPHTHALMISCHE VORRICHTUNG
APPAREIL OPHTALMIQUE

(30) Priority: 29.09.2016 JP 2016191816
(43) Date of publication of application: 04.04.2018
(73) Proprietor: Nidek Co., Ltd., Gamagori-shi, Aichi, 443-0038 (JP)
(72) Inventor: KATO, Hisatoshi, Gamagori-shi, Aichi 443-0038 (JP); SUZUKI, Nobuo, Gamagori-shi, Aichi 443-0038 (JP)
(74) Representative: Prüfer & Partner mbB Patentanwälte · Rechtsanwälte

(56) References cited:
- WO-A1-2008/030872
- US-A1- 2013 150 782
- US-B2- 9 005 157

## Description

### BACKGROUND

The present disclosure relates to an ophthalmic apparatus to be used in ophthalmic surgery.

US 9 005 157 B2 (Patent Document 1) discloses a surgical cassette device that can switch a pump to be connected to a handpiece between a peristaltic pump and a venturi pump by use of a selector.

WO 2008/030872 A1 discloses a system and method for power and flow rate control for aspiration. The document discloses an irrigation/aspiration cassette including a handpiese, a holding tank, a vacuum-based pump, a selection valve, a flow pump and a collection bag. The document does not disclose any path detouring the holding tank. Further, the document does not disclose that the flow pump discharges and deliver the fluid from the holding tank into the collection bag as well as to aspirate the fluid from the handpiece and directly deliver it to the collection bag.

US 2013/150782 A1 discloses a selective movable valve elements for aspiration and irrigation circuits including a handpiece, a peristaltic pump, a drain line reservoir, a drain bag, an aspiration exhaust line fluidly connected to the drain line reservoir, a further exhaust line fluidly connected directly to drain bag. The document does not disclose any pump which aspirate air from the drain line reservoir. Further, the document does not disclose any pump which discharges and deliver the fluid from the drain line reservoir into the drain bag as well as to aspirate the fluid from the handpiece and directly deliver it to the drain bag.

### SUMMARY

In the surgical cassette device disclosed in Patent Document 1, the peristaltic pump and the venturi pump are provided in separate paths so that the pumps are selectively connected to the handpiece by the selector. While one of the pumps is in use, the other pump is at rest. Therefore, at the time of switching the pump to the other one for connection to the handpiece, the aspiration pressure at an aspiration port of the handpiece may change because of differences in characteristic between the pumps. It is thus difficult to freely switch the pumps during aspiration.

The present disclosure has been made to address the above problems and has a purpose to provide an ophthalmic apparatus that can switch an aspiration part (a pump) without interruption or sudden change in the aspiration pressure and thus is easy to operate for a surgeon to efficiently perform surgery.

The above mentioned purpose is achieved by the ophthalmic apparatus according to claim 1.

Further developments of the present invention are given in the dependent claims.

According to the ophthalmic apparatus of the present disclosure, an aspiration part can be switched to another one without interruption or sudden change in aspiration pressure during aspiration, which is thus easy for a surgeon to operate to efficiently perform surgery.

### BRIEF DESCRIPTION OF THE DRAWINGS

FIG. 1 is an external view of anophthalmic apparatus in an embodiment;
FIG. 2 is a schematic structural view of the ophthalmic apparatus in FIG. 1 and illustrates a first aspiration mode;
FIG. 3 is a schematic structural view for explanation of a control unit of the ophthalmic apparatus in FIG. 1; and
FIG. 4 is a schematic structural view of the ophthalmic apparatus in FIG. 1 and illustrates a second aspiration mode.

### DETAILED DESCRIPTION OF THE EXEMPLARY EMBODIMENTS

An ophthalmic apparatus 1 in an embodiment of this disclosure will be explained below. This ophthalmic apparatus 1 is used for example in cataract vitreous surgery. In the present embodiment, as will be described in detail later, an aspiration mode of aspirating waste fluid LQW from a patient's eye E is switchably selected from between a first aspiration mode in which a first aspiration part 29 serves as an aspiration source for aspirating the waste fluid LQW from the patient's eye E and a second aspiration mode in which a second aspiration part 31 serves as an aspiration source for aspirating the waste fluid LQW from the patient's eye E. Therefore, the whole structure of the ophthalmic apparatus 1 will be described first, and subsequently the aspiration modes in the present embodiment will be explained.

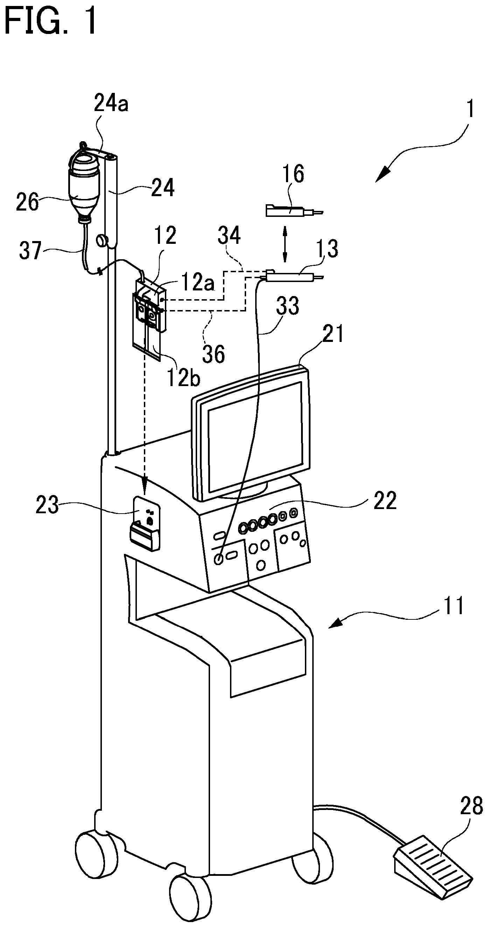

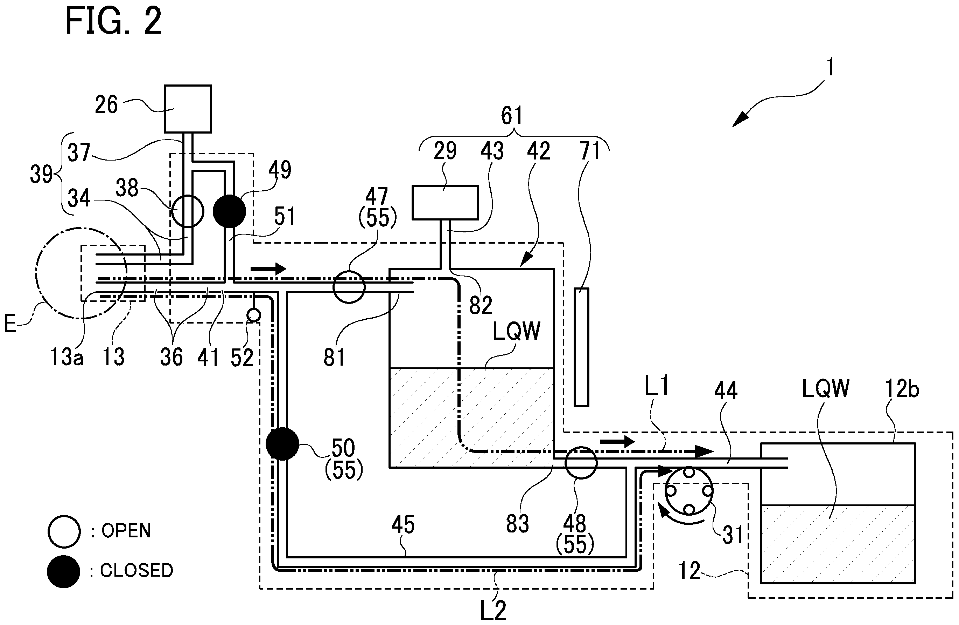

As shown in FIGs. 1 and 2, the ophthalmic apparatus 1 includes, as one example, a main unit 11 and a cassette 12. The main unit 11 is provided with a monitor 21 and a connection panel 22. The monitor 21 displays, as one example, a screen for setting surgical conditions, a list of surgical results corresponding to drive results of the surgical apparatus, and others. On the connection panel 22, as one example, there are provided a plurality of connectors to which a cable of a surgical handpiece 13 or other cables are connected.

The main unit 11 is provided, as one example, with a holder unit 23 for holding the cassette 12 for cataract surgery or others.

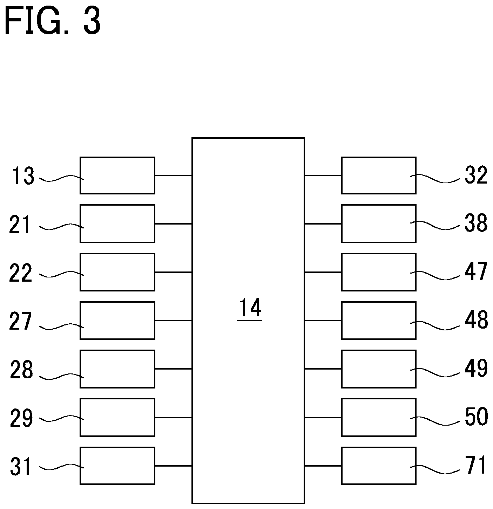

The main unit 11 is provided with a pole 24 and an arm 24a on a back face of the unit 11. The pole 24 supports an irrigation bottle 26 that contains an irrigation fluid such as normal saline solution. This bottle 26 is hung from the arm 24a horizontally extending from the top of the pole 24. The pole 24 can be moved vertically, or changed in height, by driving of a drive mechanism 27 (see FIG. 3) controlled by a control unit 14 (see FIG. 3).

The main unit 11 is further provided with an interface part on the back face. The interface part may be connected, for example, to external devices (PC) via a LAN, a USB storage device, etc. in order to externally transfer electronic data representing the surgical results and others.

The main unit 11 includes a footswitch 28. This footswitch 28 may be used to adjust various operations, such as supersonic vibration and aspiration, of a movable tip provided at a distal end of the surgical handpiece 13.

The main unit 11 internally includes the control unit 14. This control unit 14 is used, as one example, to control various operations of the ophthalmic apparatus 1.

As shown in FIG. 3, to the control unit 14, there are connected for example the surgical handpiece 13, the monitor 21, the connection panel 22, the drive mechanism 27, the footswitch 28, the first aspiration part 29, the second aspiration part 31, a storage part 32, an irrigation valve part 38, a first valve 47, a second valve 48, a vent valve part 49, a third valve 50, a fluid level measuring part 71, and the interface part. The storage part 32 may be for example a rewritable flash memory. This storage part 32 may store programs to perform surgery and various surgical information obtained in surgical operations of the main unit 11.

The control unit 14 reads out for example an irrigation aspiration program stored in the storage part 32. The control unit 14 serves as a processor for controlling driving of the first aspiration part 29, driving of the second aspiration part 31, and driving of various valves and various valve parts. The processor also may be selected from among a CPU (microprocessor), a DSP (digital signal processor), a PLD (programmable logic device), and others.

### <Surgical Handpiece>

The ophthalmic apparatus 1 may include the surgical handpiece 13. The ophthalmic apparatus 1 uses an ultrasonic (US) handpiece as one example of the surgical handpiece 13. This US handpiece is configured to emulsify and aspirate the opaque lens nucleus having become less transparent and hardened because of a cataract by ultrasonically vibrating a cutting tip provided at the distal end of the handpiece. For this ultrasonic vibration of the cutting tip, electric power may be supplied to an ultrasonic transducer provided in the surgical handpiece 13 via a power cable 33. The surgical handpiece 13 is not limited to the US handpiece. For example, the surgical handpiece 13 also may be an I/A handpiece 16 (an irrigation aspirating handpiece). The surgical handpiece 13 in the present example is connected to an irrigation tube 34 and an aspiration tube 36 in addition to the power cable 33. The irrigation tube 34 is used for example to deliver the irrigation fluid into the patient's eye E. The aspiration tube 36 is used for example to aspirate waste fluid LQW containing intraocular tissues and irrigation fluid out of the patient's eye E.

### <Irrigation Fluid Supplying Part>

The ophthalmic apparatus 1 in the present example includes an irrigation fluid supplying part. This irrigation fluid supplying part supplies e.g. the irrigation fluid to the patient's eye E. The irrigation fluid supplying part is provided, as one example, with the drive mechanism 27, the pole 24, the irrigation bottle 26, an irrigation tube 37, the irrigation tube 34, the irrigation valve part 38, and the surgical handpiece 13. Further, the irrigation fluid supplying part includes an irrigation passage 39 through which the irrigation fluid flows. This irrigation passage 39 includes the irrigation tube 34 and the irrigation tube 37. As described above, the irrigation bottle 26 filled with the irrigation fluid is hung from the pole 24 of the ophthalmic apparatus 1 in the present embodiment. The pole 24 is moved up and down, changing its height, by driving of the drive mechanism 27 controlled by the control unit 14. This upward/downward movement of the pole 24 adjusts the supply pressure of the irrigation fluid allowed to flow from the irrigation bottle 26. The irrigation fluid flowing from the irrigation bottle 26 passes through the irrigation tube 37 and the irrigation tube 34 and is irrigated into the patient's eye E via the surgical handpiece 13 grasped by a surgeon. The outflow of irrigation fluid flowing through the irrigation passage 39 is controlled at some place in the irrigation passage 39.

### <Aspiration Mechanism>

The ophthalmic apparatus 1 in the present embodiment includes an aspiration mechanism. The aspiration mechanism aspirates the waste fluid LQW including the intraocular tissues and the irrigation fluid out of the patient's eye E by use of the first aspiration part 29 or the second aspiration part 31. This aspiration mechanism is provided, as one example, with the surgical handpiece 13, the aspiration tube 36, a drain passage 41, a tank 42 (a reservoir part), an air aspiration passage 43, the first aspiration part 29, a drain passage 44, the second aspiration part 31, a drain passage 45, and a drain bag 12b (a drain part).

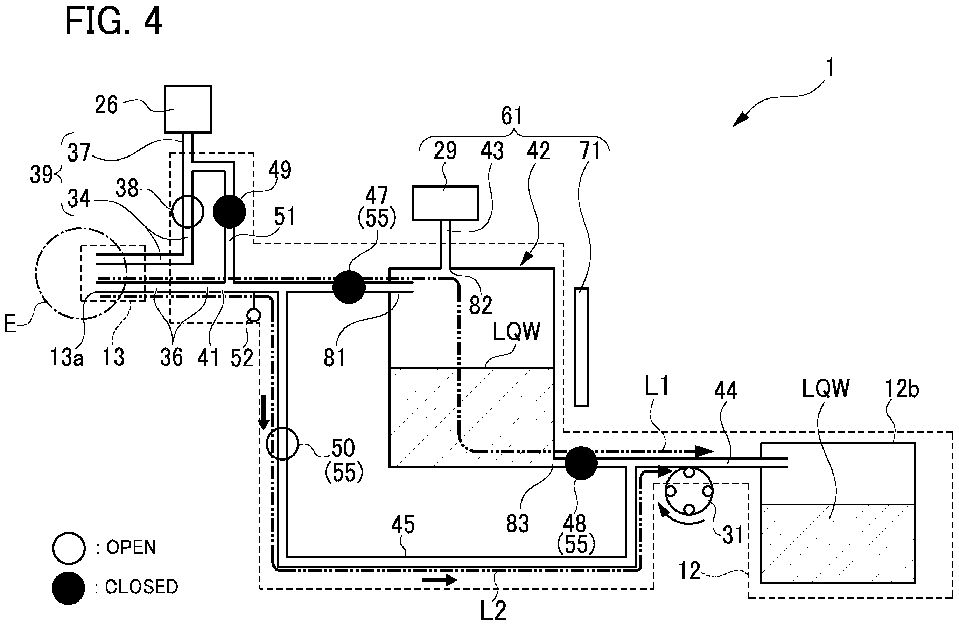

The drain passage 41 is a flow channel in which the waste fluid LQW aspirated from the patient's eye E flows. This drain passage 41 is connected to the aspiration tube 36 and the tank 42. In the present embodiment, in the drain passage 41, the first valve 47 is placed. Thus, in the first path L1 (see FIGs. 2 and 4), at least part of a switching part 55 (see FIGs. 2 and 4) is placed in a position upstream of the tank 42. The control unit 14 controls the first valve 47 to open or close, so that the drain passage 41 is opened or closed at a portion in which the first valve 47 is placed. Furthermore, the drain passage 41 is provided with a pressure sensor 52 for detecting the internal pressure of the drain passage 41.

The tank 42 temporarily stores the waste fluid LQW aspirated out of the patient's eye E by the surgical handpiece 13. The air aspiration passage 43 is connected to the tank 42 and the first aspiration part 29. The drain passage 41 and the air aspiration passage 43 are connected to the tank 42, above the fluid level of the waste fluid LQW stored in the tank 42.

The first aspiration part 29 generates an aspiration force to suck the air out of the tank 42. For instance, the control unit 14 can adjust the aspiration force of the first aspiration part 29. A venturi pump is used as one example of the first aspiration part 29. The first aspiration part 29 may be for example a vacuum pump (a vacuum generator). As the first aspiration part 29 aspirates the air from the tank 42, the pressure of air in the tank 42 decreases, thereby drawing the waste fluid LQW into the tank 42.

The drain passage 44 is connected to the tank 42 and the drain bag 12b. The drain passage 44 in the present embodiment is connected to the tank 42, below the fluid level of the waste fluid LQW stored in the tank 42. In the drain passage 44, the second valve 48 is placed. Further, the control unit 14 controls the second valve 48 to open or close, so that the drain passage 44 is opened or closed at a portion in which the second valve 48 is placed. In the present example at least a part of the drain passage 44 is made of an elastic member. The second aspiration part 31 in the present embodiment is placed at the side of the drain passage 44. The aforementioned elastic member is squeezed by a squeezing member of the second aspiration part 31 to generate the aspiration force to aspirate the waste fluid LQW.

In the present embodiment, the aspiration force of the second aspiration part 31 is used to deliver the waste fluid LQW stored in the tank 42 to the drain bag 12b. In the present embodiment, a peristaltic pump is used as one example of the second aspiration part 31. As alternatives, the second aspiration part 31 may also be selected from for example a scroll pump, a vane pump, a diaphragm pump, and other pumps. The drain bag 12b collects the waste fluid LQW delivered from the second aspiration part 31. In the present embodiment, the drain passage 44 is connected to the drain bag 12b, above the fluid level of the waste fluid LQW stored in the drain bag 12b.

The drain passage 45 is a flow channel in which the waste fluid LQW aspirated from the patient's eye E flows. The drain passage 45 is connected to the drain passage 41 and the drain passage 44. To be specific, one end of the drain passage 45 is connected to a portion of the drain passage 41, located closer to the surgical handpiece 13 than the first valve 47. The other end of the drain passage 45 is connected to a portion of the drain passage 44, located between the second valve 48 and the second aspiration part 31.

In the drain passage 45, the third valve 50 is placed. The control unit 14 controls the third valve 50 to open or close, so that the drain passage 45 is opened or closed at a portion in which the third valve 50 is placed.

In the present embodiment, the ophthalmic apparatus 1 includes a first path L1 and a second path L2 as pathways for delivering the waste fluid LQW from the aspiration port 13a of the surgical handpiece 13. The aspiration port 13a is an opening through which the waste fluid LQW is aspirated out of the patient's eye E. Specifically, for example, it is a bore of a cutting tip provided at the distal end of the surgical handpiece 13.

The first path L1 extends from the aspiration port 13a to the drain bag 12b through the tank 42. Concretely, the first path L1 is formed by the aspiration tube 36, the drain passage 41, the tank 42, and the drain passage 44. The second path L2 extends from the aspiration port 13a to the drain bag 12b by detouring around the tank 42. Specifically, the second path L2 is formed by the aspiration tube 36, the drain passage 41, the drain passage 45, and the drain passage 44.

The second aspiration part 31 in the present embodiment is connected to both the first path L1 and the second path L2 and operates to aspirate the waste fluid LQW delivered to the first path L1 and the second path L2. The pathway for connecting the aspiration port 13a to the second aspiration part 31 is switched between the first path L1 and the second path L2 by the switching part 55 constituted of the first valve 47, the second valve 48, and the third valve 50. The first valve 47 and the second valve 48 correspond to first-path opening/closing means to open/close the first path L1. The third valve 50 corresponds to second-path opening/closing means to open/close the second path L2. In the present example the control unit 14 controls the first valve 47 and the second valve 48 to be set in the open/closed states different from the third valve 50 to thereby switch the pathway for connecting the aspiration port 13a to the second aspiration part 31 to the first path L1 or the second path L2. The aspiration modes for performing aspiration using the above configured aspiration mechanism will be described later.

### <Vent Flow Passage>

The ophthalmic apparatus 1 in the present example includes a vent flow passage 51. This vent flow passage 51 is connected to the irrigation passage 39 and the drain passage 41. Flowing of the irrigation fluid in the vent flow passage 51 suppresses excessive aspiration from the patient's eye E. The vent flow passage 51 includes the vent valve part 49. The control unit 14 controls the vent valve part 49 to open and close, thereby controlling a flow rate of the irrigation fluid allowed to flow in the vent flow passage 51.

### <Cassette>

The cassette 12 in the present example includes the cassette body 12a and the drain bag 12b. To the cassette body 12a in the present example there are connected the irrigation tube 34, the irrigation tube 37, and the aspiration tube 36. The cassette body 12a includes, for example, the drain passage 41, the drain passage 44, the drain passage 45, and the air aspiration passage 43. The drain bag 12b is placed outside the casing of the cassette body 12a.

### <Fluid Reservoir Device>

Next, a drain reservoir device 61 will be described. As shown in FIG. 2, the drain reservoir device 61 includes the tank 42, the air aspiration passage 43, the first aspiration part 29, the fluid level measuring part 71, and others.

The tank 42 is a reservoir part for temporarily storing the waste fluid LQW aspirated from the patient's eye E. As shown in FIG. 2, the tank 42 is placed on an upstream side of the drain bag 12b and is provided with a drain inlet 81, an air outlet 82, and a drain outlet 83.

The drain inlet 81 is an opening which is connected to the drain passage 41 and through which the waste fluid LQW is drawn from the drain passage 41 into the tank 42. The air outlet 82 is an opening which is continuous with the air aspiration passage 43 and through which the air in the tank 42 is discharged from the tank 42. The drain outlet 83 is an opening which is continuous with the drain passage 44 and through which the waste fluid LQW stored in the tank 42 is discharged from the tank 42.

The fluid level measuring part 71 measures the fluid level of the waste fluid LQW stored in the tank 42. Specifically, the fluid level measuring part 71 in the present example measures the fluid level of the waste fluid LQW in the tank 42 through a transparent window (not shown) provided in the tank 42. A measurement result of the fluid level measuring part 71 is transmitted to the control unit 14 (see FIG. 3). The fluid level measuring part 71 is constituted of for example a light emitting part and a light receiving part (e.g. a linear image sensor).

In the drain reservoir device 61 configured as above, the air is discharged from the tank 42 through the air outlet 82 into the air aspiration passage 43 by aspiration of the first aspiration part 29. Thus, the pressure in the tank 42 decreases. Accordingly, the waste fluid LQW extracted from the patient's eye E is drawn into and stored in the tank 42 through the aspiration tube 36, the drain passage 41, and the drain inlet 81. Simultaneously, the waste fluid LQW is caused to drain from the tank 42 into the drain passage 44 through the drain outlet 83 by the second aspiration part 31 at a flow rate of the waste fluid LQW regulated based on the measurement result of the fluid level of the waste fluid LQW in the tank 42 measured by the fluid level measuring part 71 so that the fluid level of the waste fluid LQW in the tank 42 is held constant. In this manner, on an upstream side of the drain bag 12b, the tank 42 is provided to temporarily store the waste fluid LQW aspirated from the patient's eye E by aspiration of the first aspiration part 29. This can improve responsiveness in aspirating the waste fluid LQW from the patient's eye E.

### <Aspiration Mode>

Next, an aspiration mode using the aforementioned irrigation fluid supply part and aspiration mechanism will be described below. The aspiration mode in the present example includes a first aspiration mode and a second aspiration mode. Herein, the first aspiration mode is a mode in which the first aspiration part 29 is used as an aspiration source for aspirating the waste fluid LQW from the patient's eye E. Further, the second aspiration mode is a mode in which the second aspiration part 31 is used as an aspiration source for aspirating the waste fluid LQW from the patient's eye E.

The first aspiration mode is explained first. In this first aspiration mode, the control unit 14 causes the first valve 47 and the second valve 48 to open and the third valve 50 to close, as shown in FIG. 2. At that time, the control unit 14 causes the irrigation valve part 38 to open and the vent valve part 49 to close. Accordingly, the aspiration port 13a is brought into communication with the first aspiration part 29 through the aspiration tube 36, the drain passage 41, the tank 42, and the air aspiration passage 43. Furthermore, at that time, the aspiration port 13a is also brought into communication with the second aspiration part 31 through the first path L1 formed by the aspiration tube 36, the drain passage 41, the tank 42, and the drain passage 44. In the first aspiration mode, as above, the switching part 55 sets the first path L1 as the pathway connecting the aspiration port 13a to the second aspiration part 31.

The control unit 14 causes the first aspiration part 29 to aspirate the air from the tank 42 through the air aspiration passage 43. Thus, the air pressure in the tank 42 decreases, thereby allowing the waste fluid LQW to be aspirated from the patient's eye E into the tank 42 through the aspiration port 13a, the aspiration tube 36, and the drain passage 41, and is stored in the tank 42.

Simultaneously, the control unit 14 causes the second aspiration part 31 to aspirate the waste fluid LQW from the tank 42 to deliver the waste fluid LQW into the drain bag 12b through the drain passage 44. More specifically, the control unit 14 causes the second aspiration part 31 to draw and discharge, from the tank 42, the waste fluid LQW in an amount equal to the amount of the waste fluid LQW aspirated into the tank 42 by the first aspiration part 29. Accordingly, the storage amount of the waste fluid LQW in the tank 42 is kept constant. The fluid level of the waste fluid LQW stored in the tank 42 is being measured by the fluid level measuring part 71 of the drain reservoir device 61 as described above.

Thus, in the first aspiration mode, the waste fluid LQW is delivered from the patient's eye E to the aspiration port 13a, the aspiration tube 36, the drain passage 41, the tank 42, the drain passage 44, and the second aspiration part 31 in sequence, and finally into the drain bag 12b. In the first aspiration mode, specifically, the first aspiration part 29 aspirates and delivers the waste fluid LQW from the patient's eye E into the tank 42, and the second aspiration part 31 aspirates and delivers the waste fluid LQW from the tank 42 into the drain bag 12b. In the first aspiration mode, therefore, the second aspiration part 31 is used to draw the waste fluid LQW stored in the tank 42 and deliver this drawn waste fluid LQW into the drain bag 12b.

Next, the second aspiration mode will be described. In this second aspiration mode, as shown in FIG. 4, the control unit 14 causes the first valve 47 and the second valve 48 to close and the third valve 50 to open. In this state, the control unit 14 keeps the irrigation valve part 38 open and the vent valve part 49 closed. Accordingly, the aspiration port 13a is brought into communication with the second aspiration part 31 through the second path L2 formed by the aspiration tube 36, the drain passage 41, the drain passage 45, and the drain passage 44. The control unit 14 causes the second aspiration part 31 to draw the waste fluid LQW from the aspiration port 13a through the drain passages 44, 45, and 41 and the aspiration tube 36.

In the second aspiration mode, as described above, the waste fluid LQW is delivered from the patient's eye E to the aspiration port 13a, aspiration tube 36, drain passage 41, drain passage 45, drain passage 44, and second aspiration part 31 in sequence, and finally into the drain bag 12b. In the second aspiration mode, therefore, the second aspiration part 31 is used to aspirate the waste fluid LQW from the patient's eye E and directly deliver this aspirated waste fluid LQW into the drain bag 12b, that is, used as an aspiration source for aspirating the waste fluid LQW from the patient's eye E.

In the present embodiment, as described above, the second aspiration part 31 is configured to serve two intended purposes; one is to draw the waste fluid LQW stored in the tank 42 and deliver it to the drain bag 12b and the other is to aspirate the LQW from the patient's eye E and directly deliver it to the drain bag 12b.

In the present embodiment, the first aspiration mode and the second aspiration mode can be switched, or selectively used, in the course of surgery. In other words, during surgery, the pathway for connecting the aspiration port 13a to the second aspiration part 31 is switched between the first path L1 and the second path L2, so that the aspiration source for aspirating the waste fluid LQW from the patient's eye E can be changed from the first aspiration part 29 to the second aspiration part 31, or vice versa. This switching between the first aspiration mode and the second aspiration mode can be performed manually by a surgeon (e.g., by operation on a selection screen of the monitor 21 by the surgeon) or automatically by the control unit 14.

Herein, as described above, in the first aspiration mode (see FIG. 2), the control unit 14 controls the first aspiration part 29 to aspirate the waste fluid LQW from the patient's eye E and simultaneously controls the second aspiration part 31 to deliver the waste fluid LQW from the tank 42 to the drain bag 12b. To be concrete, the control unit 14 drives the second aspiration part 31 to discharge the waste fluid LQW from the tank 42 in an amount equal to the inflow amount of the waste fluid LQW flowing in the tank 42.

In the first aspiration mode, as above, the second aspiration part 31 is operated according to the amount of the waste fluid LQW aspirated into the tank 42 through the aspiration port 13a by the first aspiration part 29 and further according to the aspirating condition at the aspiration port 13a. Herein, the aspirating condition at the aspiration port 13a varies, from moment to moment during surgery, according to the conditions of aspirated substances in the aspiration port 13a, such as the size and the shape of intraocular tissues and the viscosity of the waste fluid LQW aspirated into the aspiration port 13a. For instance, the aspiration pressure is relatively stable when the waste fluid LQW with low viscosity is aspirated into the aspiration port 13a. In contrast, the aspiration pressure tends to become high when the waste fluid LQW with high viscosity is aspirated into the aspiration port 13a or when large intraocular tissues occlude the aspiration port 13a. In the present embodiment, in the first aspiration mode, the second aspiration part 31 is driven by changing the aspiration pressure of the second aspiration part 31 according to the ever-changing aspirating condition in the aspiration port 13a as above.

In the present example at the time of switching the aspiration mode from the first aspiration mode to the second aspiration mode, the control unit 14 controls the switching part 55 to change the pathway for connecting the aspiration port 13a to the second aspiration part 31 from the first path L1 to the second path L2 to thereby switch the aspiration source for aspirating the waste fluid LQW from the patient's eye E to the second aspiration part 31, while maintaining the operation (aspiration pressure) of the second aspiration part 31, e.g., the number of rotations of a squeezing member of the second aspiration part 31.

Accordingly, while maintaining the operation of the second aspiration part 31 according to the ever-changing aspirating condition in the aspiration port 13a, the aspiration source for aspirating the waste fluid LQW from the patient's eye E can be switched from the first aspiration part 29 to the second aspiration part 31. For instance, even if the aspiration source is switched to the second aspiration part 31 while the intraocular tissues are held in the aspiration port 13a by aspiration of the first aspiration part 29, it is possible to keep the intraocular tissues held in the aspiration port 13a by aspiration of the second aspiration part 31 while maintaining the aspirating state. Thereafter, the aspiration pressure of the second aspiration part 31 is adjusted, so that the intraocular tissues can be delivered from the aspiration port 13a to the drain bag 12b. In this manner, the aspiration source for aspirating the waste fluid LQW from the patient's eye E can be switched from the first aspiration part 29 to the second aspiration part 31 without interruption or sudden change in the aspiration pressure in the aspiration port 13a.

In the present example in the second aspiration mode (see FIG. 4), the control unit 14 controls the second aspiration part 31 to aspirate the waste fluid LQW from the patient's eye E and simultaneously controls the first aspiration part 29 to adjust the internal pressure of the tank 42 to be equal to the aspiration pressure of the second aspiration part 31. To be concrete, the control unit 14 controls the first aspiration part 29 adjust the internal pressure of the tank 42 to be equal to the pressure detected by the pressure sensor 52 so that the internal pressure of the tank 42 is equal to the aspiration pressure of the second aspiration part 31 (i.e. the aspiration pressure in the aspiration port 13a). At that time, since the first valve 47 is in a closed state, the waste fluid LQW delivered to the drain passage 41 is not drawn into the tank 42 even if the first aspiration part 29 is activated.

In the present example in switching the second aspiration mode to the first aspiration mode as described above, the control unit 14 causes the switching part 55 to switch the pathway for connecting the aspiration port 13a to the second aspiration part 31, from the second path L2 to the first path L1, to thereby change the aspiration source for aspirating the waste fluid LQW out of the patient's eye E to the first aspiration part 29 while maintaining the internal pressure of the tank 42. In the above manner, when the pathway for connecting the aspiration port 13a to the second aspiration part 31 is switched from the second path L2 to the first path L1 by the switching part 55, the control unit 14 controls the first aspiration part 29 to adjust the internal pressure of the tank 42 to be equal to the aspiration pressure in the aspiration port 13a.

Accordingly, it is possible to switch over the aspiration source for aspirating the waste fluid LQW out of the patient's eye E from the second aspiration part 31 to the first aspiration part 29 according to the ever-changing aspirating condition in the aspiration port 13a. Thus, switching of the aspiration source for aspirating the waste fluid LQW out of the patient's eye E can be switched from the second aspiration part 31 to the first aspiration part 29 without interruption or sudden change in the aspiration pressure in the aspiration port 13a.

As described above, the aspiration source for aspirating the waste fluid LQW from the patient's eye E can be switched from the first aspiration part 29 to the second aspiration part 31 or from the second aspiration part 31 to the first aspiration part 29 without interruption or sudden change in the aspiration pressure in the aspiration port 13a. Therefore, according to the aspirating condition at different times in one aspiration operation during surgery, a surgeon can freely select a preferable aspiration part as the aspiration source for aspirating the waste fluid LQW from the patient's eye E. Thus, the ophthalmic apparatus 1 is easy for a surgeon to use to efficiently perform surgery.

In the second aspiration mode, for instance, the control unit 14 may stop the first aspiration part 29 in advance and then activate the first aspiration part 29 just before or concurrently with switching the second aspiration mode to the first aspiration mode. In this case, when causing the switching part 55 to switch the pathway for connecting the aspiration port 13a to the second aspiration part 31, from the second path L2 to the first path L1, the control unit 14 controls the first aspiration part 29 to adjust the internal pressure of the tank 42 to be equal to the aspiration pressure in the aspiration port 13a. Thus, power consumption can be reduced.

The ophthalmic apparatus in the present embodiment includes: the aspiration port 13a through which the waste fluid LQW is aspirated from the patient's eye E, the tank 42 for storing the waste fluid LQW aspirated through the aspiration port 13a, the first aspiration part 29 configured to aspirate air from the tank 42 to decrease the internal pressure of the tank 42 to thereby draw the waste fluid LQW into the tank 42, the first path L1 extending from the aspiration port 13a to the drain bag 12b through the tank 42, the second path L2 extending from the aspiration port 13a to the drain bag 12b by detouring around the tank 42, the second aspiration part 31 connected to the first path L1 and the second path L2 and configured to aspirate the waste fluid LQW delivered to the first path L1 and the second path L2, the drain bag 12b for collecting the waste fluid LQW delivered from the second aspiration part 31, and the switching part 55 configured to switch the pathway for connecting the aspiration port 13a to the second aspiration part 31 between the first path L1 and the second path L2.

In the present embodiment, as described above, the second aspiration part 31 serves two intended purposes; one is to discharge the waste fluid LQW from the tank 42 and deliver it to the drain bag 12b and the other is to aspirate the waste fluid LQW from the patient's eye E and directly deliver it to the drain bag 12b. Thus, the aspiration source for aspirating the waste fluid LQW from the patient's eye E can be changed over to the first aspiration part 29 or the second aspiration part 31. Further, for instance, when the first aspiration part 29 is set as the aspiration source for aspirating the waste fluid LQW from the patient's eye E, the second aspiration part 31 may remain operating to discharge the waste fluid LQW out of the tank 42 into the drain bag 12b. Accordingly, the aspiration source for aspirating the waste fluid LQW from the patient's eye E can be switched from the first aspiration part 29 to the second aspiration part 31 without interruption or sudden change in the aspiration pressure in the aspiration port 13a. Moreover, since the internal pressure of the tank 42 is controlled by the first aspiration part 29, the aspiration source for aspirating the waste fluid LQW from the patient's eye E can be switched from the second aspiration part 31 to the first aspiration part 29 without interruption or sudden change in the aspiration pressure in the aspiration port 13a.

According to the ophthalmic apparatus 1 in the present example irrespective of differences in aspirating condition in the aspiration port 13a, the aspiration source for aspirating the waste fluid LQW from the patient's eye E can be switched to the first aspiration part 29 or the second aspiration part 31 without interruption or sudden change in the aspiration pressure. Accordingly, a surgeon can freely select a preferable aspiration part as the aspiration source for aspirating the waste fluid LQW from the patient's eye E according to not only differences in type of surgery and steps for surgery but also the aspirating conditions in the aspiration port 13a at different times in one aspiration operation during surgery. For instance, a surgeon can choose one from the first aspiration part 29 and the second aspiration part 31 which are different in responsiveness of the aspiration pressure, according to the aspirating conditions in the aspiration port 13a. The ophthalmic apparatus 1 therefore, allows the aspiration part to be changed over without interruption or sudden change in the aspiration pressure during aspiration and is easy for a surgeon to use to efficiently perform surgery.

Since the second aspiration part 31 is configured to serve two intended purposes; one is to discharge and deliver the waste fluid LQW from the tank 42 into the drain bag 12b and the other is to aspirate the waste fluid LQW from the patient's eye E and directly deliver it to the drain bag 12b. Thus, the single second aspiration part 31 suffices for the aforementioned operations. This can reduce the manufacturing cost of the ophthalmic apparatus 1.

While the first path L1 is set as the pathway for connecting the aspiration port 13a to the second aspiration part 31 by the switching part 55, the control unit 14 controls the second aspiration part 31 to deliver the waste fluid LQW from the tank 42 to the drain bag 12b. On the other hand, at the time when the pathway for connecting the aspiration port 13a to the second aspiration part 31 is switched from the first path L1 to the second path L2, the control unit 14 controls the second aspiration part 31 to continue to operate. Accordingly, the aspiration source for aspirating the waste fluid LQW from the patient's eye E can be changed over from the first aspiration part 29 to the second aspiration part 31 without interruption or sudden change in the aspiration pressure in the aspiration port 13a.

When the pathway for connecting the aspiration port 13a to the second aspiration part 31 is switched from the second path L2 to the first path L1 by the switching part 55, the control unit 14 controls the first aspiration part 29 to adjust the internal pressure of the tank 42 to be equal to the aspiration pressure in the aspiration port 13a. Accordingly, the aspiration source for aspirating the waste fluid LQW from the patient's eye E can be smoothly changed over from the second aspiration part 31 to the first aspiration part 29 without interruption or sudden change in the aspiration pressure in the aspiration port 13a.

The foregoing embodiments are mere examples and give no limitation to the present disclosure and obviously various modifications and changes are possible without departing from the essential characteristics thereof.

For instance, the switching part 55 also may be a proportional valve that can be set to an intermediate opening degree in addition to full open and full closed degrees. Further, a peristaltic pump may be additionally provided as a special aspiration source for aspirating the waste fluid LQW from the patient's eye E in addition to the first aspiration part 29 and the second aspiration part 31.

### Reference Signs List

- 1: Ophthalmic apparatus
- 11: Main unit
- 12: Cassette
- 12a: Cassette body
- 12b: Drain bag
- 13: Surgical handpiece
- 13a: Aspiration port
- 14: Control unit
- 21: Monitor
- 29: First aspiration part
- 31: Second aspiration part
- 36: Aspiration tube
- 41: Drain passage
- 42: Tank
- 43: Air aspiration passage
- 44: Drain passage
- 45: Drain passage
- 47: First valve
- 48: Second valve
- 50: Third valve
- 52: Pressure sensor
- 55: Switching part
- E: Patient's eye
- LQW: Waste fluid
- L1: First path
- L2: Second path

## Claims

1. An ophthalmic apparatus (1) comprising:
an aspiration port (13a) through which a fluid including intraocular tissues is aspirated from a patient's eye (E);
a reservoir part (42) for storing the fluid including the intraocular tissues aspirated through the aspiration port (13a);
a first aspiration part (29) configured to aspirate air from the reservoir part (42) to decrease internal pressure of the reservoir part (42) to draw the fluid including the intraocular tissues into the reservoir part (42);
a first path (L1) extending from the aspiration port (13a) through the reservoir part (42);
a second path (L2) extending from the aspiration port by detouring around the reservoir part;
a second aspiration part (31) connected to the first path (L1) and the second path (L2) and configured to aspirate the fluid including the intraocular tissues delivered to the first path (L1) and the second path (L2);
a drain part (12b) for collecting the fluid including the intraocular tissues delivered from the second aspiration part (31); and
a switching part (55) configured to switch a pathway for connecting the aspiration port (13a) to the second aspiration part (31) between the first path (L1) and the second path (L2);
wherein the second aspiration part (31) is configured to discharge and deliver the fluid including the intraocular tissues from the reservoir part (42) into the drain part (12b) and to aspirate the fluid including the intraocular tissues from the patient's eye (E) and
directly deliver it to the drain part (12b).

2. The ophthalmic apparatus (1) according to claim 1, wherein
when the first path (L1) is set as the pathway for connecting the aspiration port (13a) to the second aspiration part (31) by the switching part (55), the second aspiration part (31) is configured to deliver the fluid including the intraocular tissues stored in the reservoir part (42) to the drain part (12b), and
when the pathway for connecting the aspiration port (13a) to the second aspiration part (31) is switched from the first path (L1) to the second path (L2) by the switching part (55), the second aspiration part (31) is configured to continue to operate.

3. The ophthalmic apparatus (1) according to claim 1 or 2, wherein when the pathway for connecting the aspiration port (13a) to the second aspiration part (31) is switched from the second path (L2) to the first path (L1) by the switching part (55), the first aspiration part (29) is configured to adjust the internal pressure of the
reservoir part (42) to be equal to aspiration pressure in the aspiration port (13a).

4. The ophthalmic apparatus (1) according to any one of claims 1 to 3, wherein
when the pathway for connecting the aspiration port (13a) to the second aspiration part (31) is set to the second path (L2) by the switching part (55), the first aspiration part (29) is configured to adjust the internal pressure of the reservoir part (42) to be equal to aspiration pressure of the second aspiration part.

5. The ophthalmic apparatus (1) according to any one of claims 1 to 3, wherein
when the pathway for connecting the aspiration port (13a) to the second aspiration part (31) is set to the second path (L2) by the switching part (55), the first aspiration part (29) is configured to be stopped, and
just before or concurrently with switching the pathway for connecting the aspiration port (13a) to the second aspiration part (31) from the second path (L2) to the first path (L1) by the switching part (55), the first aspiration part is configured to be activated.

6. The ophthalmic apparatus (1) according to any one of claims 1 to 5, wherein
at least part of the switching part (55) is placed in the first path (L1) in a position upstream of the reservoir part (42).

7. The ophthalmic apparatus (1) according to any one of claims 1 to 6, wherein
the switching part (55) includes first-path opening/closing means for opening/closing the first path (L1) and second-path opening/closing means for opening/closing the second path (L2), and
the first-path opening/closing means and the second-path opening/closing means are set in different open/closed states from each other to switch the pathway for connecting the aspiration port (13a) to the second aspiration part (31) to the first path (L1) or the second path (L2).

## Patentansprüche

1. Eine ophthalmische Vorrichtung (1), umfassend:
eine Ansaugöffnung (13a), durch die ein Fluid, das intraokulares Gewebe enthält, von einem Auge (E) eines Patienten angesaugt wird;
einen Reservoirteil (42) zum Speichern des Fluids einschließlich des intraokularen Gewebes, das durch die Ansaugöffnung (13a) angesaugt wurde;
einen ersten Ansaugteil (29), der so konfiguriert ist, dass er Luft aus dem Reservoirteil (42) ansaugt, um den Innendruck des Reservoirteils (42) zu verringern, um das Fluid einschließlich des intraokularen Gewebes in den Reservoirteil (42) zu saugen;
einen ersten Pfad (L1), der sich von der Ansaugöffnung (13a) durch den Reservoirteil (42) erstreckt;
einen zweiten Pfad (L2), der sich von der Ansaugöffnung über ein Umleiten um den Reservoirteil herum erstreckt;
einen zweiten Ansaugteil (31), der mit dem ersten Pfad (L1) und dem zweiten Pfad (L2) verbunden ist und so konfiguriert ist, dass er das Fluid einschließlich des intraokularen Gewebes ansaugt, das dem ersten Pfad (L1) und dem zweiten Pfad (L2) zugeführt wird;
einen Abflussteil (12b) zum Sammeln des Fluids einschließlich des intraokularen Gewebes, das von dem zweiten Ansaugteil (31) zugeführt wird; und
einen Umschaltteil (55), der so konfiguriert ist, dass er einen Pfad zum Verbinden der Ansaugöffnung (13a) mit dem zweiten Ansaugteil (31) zwischen dem ersten Pfad (L1) und dem zweiten Pfad (L2) umschaltet, wobei
der zweite Ansaugteil (31) so konfiguriert ist, dass er das Fluid einschließlich des intraokularen Gewebes aus dem Reservoirteil (42) abführt und dem Abflussteil (12b) zuführt, und dass er das Fluid einschließlich des intraokularen Gewebes aus dem Auge (E) des Patienten ansaugt und es direkt dem Abflussteil (12b) zuführt.

2. Die ophthalmische Vorrichtung (1) nach Anspruch 1, wobei
der zweite Ansaugteil (31) konfiguriert ist, um das Fluid einschließlich des intraokularen Gewebes, das in dem Reservoirteil (42) gespeichert ist, dem Abflussteil (12b) zuzuführen, wenn der erste Pfad (L1) als der Pfad zum Verbinden der Ansaugöffnung (13a) mit dem zweiten Ansaugteil (31) durch den Umschaltteil (55) eingestellt ist, und
der zweite Ansaugteil (31) konfiguriert ist, um weiter betrieben zu werden, wenn der Pfad zum Verbinden der Ansaugöffnung (13a) mit dem zweiten Ansaugteil (31) durch den Umschaltteil (55) vom ersten Pfad (L1) auf den zweiten Pfad (L2) umgeschaltet wird.

3. Die ophthalmische Vorrichtung (1) nach Anspruch 1 oder 2, wobei der erste Ansaugteil (29) konfiguriert ist, um den Innendruck des Reservoirteils (42) anzupassen, um gleich dem Ansaugdruck in der Ansaugöffnung (13a) zu sein, wenn der Pfad zum Verbinden der Ansaugöffnung (31a) mit dem zweiten Ansaugteil (31) von dem zweiten Pfad (L2) auf den ersten Pfad (L1) durch den Umschaltteil (55) umgeschaltet wird.

4. Die ophthalmische Vorrichtung (1) nach einem der Ansprüche 1 bis 3, wobei der erste Ansaugteil (29) konfiguriert ist, um den Innendruck des Reservoirteils (42) anzupassen, um gleich dem Ansaugdruck des zweiten Ansaugteils zu sein, wenn der Pfad zum Verbinden der Ansaugöffnung (31a) mit dem zweiten Ansaugteil (31) durch den Umschaltteil (55) auf den zweiten Pfad (L2) eingestellt wird.

5. Die ophthalmische Vorrichtung (1) nach einem der Ansprüche 1 bis 3, wobei
der erste Ansaugteil (29) konfiguriert ist, um gestoppt zu werden, wenn der Pfad zum Verbinden der Ansaugöffnung (31a) mit dem zweiten Ansaugteil (31) durch den Umschaltteil (55) auf den zweiten Pfad (L2) eingestellt wird, und
der erste Ansaugteil (31a) konfiguriert ist, um kurz vor oder gleichzeitig mit einem Umschalten des Pfads zum Verbinden der Ansaugöffnung (13a) mit dem zweiten Ansaugteil (31) von dem zweiten Pfad (L2) auf den ersten Pfad (L1) durch den Umschaltteil (55) aktiviert zu werden.

6. Die ophthalmische Vorrichtung (1) nach einem der Ansprüche 1 bis 5, wobei
zumindest ein Teil des Umschaltteils (55) in dem ersten Pfad (L1) in einer Position stromaufwärts des Reservoirteils (42) platziert ist.

7. Die ophthalmische Vorrichtung (1) nach einem der Ansprüche 1 bis 6, wobei
der Umschaltteil (55) Öffnungs-/Schließ-Mittel des ersten Pfads zum Öffnen/Schließen des ersten Pfads (L1) und Öffnungs-/Schließ-Mittel des zweiten Pfads zum Öffnen/Schließen des zweiten Pfads (L2) umfasst, und
die Öffnungs-/Schließ-Mittel des ersten Pfads und die Öffnungs-/Schließ-Mittel des zweiten Pfads in voneinander unterschiedlichen Öffnungs-/Schließ-Zuständen eingestellt werden, um den Weg zum Verbinden der Ansaugöffnung (13a) mit dem zweiten Ansaugteil (31) auf den ersten Pfad (L1) oder den zweiten Pfad (L2) umzuschalten.

## Revendications

1. Dispositif ophtalmique (1) comprenant :
un orifice d'aspiration (13a) à travers lequel un fluide comportant des tissus intraoculaires est aspiré à partir d'un oeil d'un patient (E) ;
une partie de réservoir (42) destinée à stocker le fluide comportant les tissus intraoculaires aspirés à travers l'orifice d'aspiration (13a) ;
une première partie d'aspiration (29) configurée de manière à aspirer l'air à partir de la partie de réservoir (42) afin de diminuer la pression interne de la partie de réservoir (42) pour aspirer le fluide comportant les tissus intraoculaires dans la partie de réservoir (42) ;
un premier trajet (L1) s'étendant à partir de l'orifice d'aspiration (13a) à travers la partie de réservoir (42) ;
un second trajet (L2) s'étendant à partir de l'orifice d'aspiration en contournant la partie de réservoir ;
une seconde partie d'aspiration (31) raccordée au premier trajet (L1) et au second trajet (L2) et configurée de manière à aspirer le fluide comportant les tissus intraoculaires délivré au premier trajet (L1) et au second trajet (L2) ;
une partie de drain (12b) destinée à collecter le fluide comportant les tissus intraoculaires délivrés à partir de la seconde partie d'aspiration (31) ; et
une partie de commutation (55) configurée de manière à commuter une voie de passage destinée à relier l'orifice d'aspiration (13a) à la seconde partie d'aspiration (31) entre le premier trajet (L1) et le second trajet (L2) ;
dans lequel
la seconde partie d'aspiration (31) est configurée de manière à décharger et à délivrer le fluide comportant les tissus intraoculaires à partir de la partie de réservoir (42) vers la partie de drain (12b) et à aspirer le fluide comportant les tissus intraoculaires à partir de l'œil du patient (E) et à le délivrer directement à la partie de drain (12b).

2. Dispositif ophtalmique (1) selon la revendication 1, dans lequel
lorsque le premier trajet (L1) est défini comme voie de passage destinée à relier l'orifice d'aspiration (13a) à la seconde partie d'aspiration (31) par la partie de commutation (55), la seconde partie d'aspiration (31) est configurée de manière à délivrer le fluide comportant les tissus intraoculaires stocké dans la partie de réservoir (42) à la partie de drain (12b), et
lorsque la voie de passage destinée à relier l'orifice d'aspiration (13a) à la seconde partie d'aspiration (31) est commutée à partir du premier trajet (L1) vers le second trajet (L2) par la partie de commutation (55), la seconde partie d'aspiration (31) est configurée afin de continuer à fonctionner.

3. Dispositif ophtalmique (1) selon la revendication 1 ou 2, dans lequel lorsque la voie de passage destinée à relier l'orifice d'aspiration (13a) à la seconde partie d'aspiration (31) est commutée du second trajet (L2) vers le premier trajet (L1) par la partie de commutation (55), la première partie d'aspiration (29) est configurée de manière à ajuster la pression interne de la partie de réservoir (42) afin de la rendre égale à la pression d'aspiration dans l'orifice d'aspiration (13a).

4. Dispositif ophtalmique (1) selon l'une quelconque des revendications 1 à 3, dans lequel
lorsque la voie de passage destinée à relier l'orifice d'aspiration (13a) à la seconde partie d'aspiration (31) est définie comme le second trajet (L2) par la partie de commutation (55), la première partie d'aspiration (29) est configurée de manière à ajuster la pression interne de la partie de réservoir (42) afin de la rendre égale à la pression d'aspiration de la seconde partie d'aspiration.

5. Dispositif ophtalmique (1) selon l'une quelconque des revendications 1 à 3, dans lequel
lorsque la voie de passage destinée à relier l'orifice d'aspiration (13a) à la seconde partie d'aspiration (31) est définie comme le second trajet (L2) par la partie de commutation (55), la première partie d'aspiration (29) est configurée de manière à être arrêtée, et
juste avant ou simultanément à la commutation de la voie de passage destinée à relier l'orifice d'aspiration (13a) à la seconde partie d'aspiration (31) à partir du second trajet (L2) vers le premier trajet (L1) par la partie de commutation (55), la première partie d'aspiration est configurée de manière à être activée.

6. Dispositif ophtalmique (1) selon l'une quelconque des revendications 1 à 5, dans lequel
au moins une partie de la partie de commutation (55) est placée sur le premier trajet (L1) dans une position en amont de la partie de réservoir (42).

7. Dispositif ophtalmique (1) selon l'une quelconque des revendications 1 à 6, dans lequel
la partie de commutation (55) comporte des moyens d'ouverture/fermeture de premier trajet destinés à ouvrir/fermer le premier trajet (L1) et des moyens d'ouverture/fermeture de second trajet destinés à ouvrir/fermer le second trajet (L2), et
les moyens d'ouverture/fermeture de premier trajet et les moyens d'ouverture/fermeture de second trajet sont placés dans des états d'ouverture/fermeture différents l'un par rapport à l'autre afin de commuter la voie de passage destinée à relier l'orifice d'aspiration (13a) à la seconde partie d'aspiration (31) sur le premier trajet (L1) ou le second trajet (L2).
